# EUROPEAN PATENT APPLICATION

(11) **EP 1 293 199 A1**
(43) Date of publication of application: **19.03.2003**
(21) Application number: 01938619.2
(22) Date of filing: 13.06.2001
(51) Int. Cl.: A61K 9/70, A61F 13/02

(54) **PLASTER**

(30) Priority: 13.06.2000 JP 2000177091
(71) Applicant: HISAMITSU PHARMACEUTICAL CO. INC., Tosu-shi, Saga-ken 841-0017 (JP)
(72) Inventor: MIZOTA, Toshiharu, Hisamitsu Pharm. Co., Inc, Shinagawa-ku, Tokyo 141-0031 (JP); TSUTSUMI, Nobuo, Hisamitsu Pharm. Co., Inc., Shinagawa-ku, Tokyo 141-0031 (JP); OOTA, Shigeo, Hisamitsu Pharm. Co., Inc., Shinagawa-ku, Tokyo 141-0031 (JP); MUTA, Kazunori, Hisamitsu Pharm. Co., Inc., Tosu-shi, Saga 841-0017 (JP)
(74) Representative: Grünecker, Kinkeldey, Stockmair & Schwanhäusser Anwaltssozietät
(86) International application number: JP0104980
(87) International publication number: WO01095889

(57) **Abstract**

A patch comprising a laminate having an expansible support and an adhesive layer laminated on the support; and a release sheet, attached onto the adhesive layer, having perforations formed with a predetermined interval;
wherein the laminate when formed into a test piece having a longer side of 20 cm and a shorter side of 5 cm exhibits a load of 0.98 to 14.71 N/5 cm in both longer and shorter side directions at the time of 50% elongation, and a 50% elongation recovery ratio of 50 to 95% in both longer and shorter side directions.

## Description

### Technical Field

The present invention relates to a patch and, more specifically, to a patch provided with a release sheet.

### Background Art

Recently, as the aging of society has been in progress, the number of people feeling pain in their shoulders, lower backs, elbows, knees, and the like has been prone to increase. In their symptomatic therapies, patches such as poultice and tape preparation are often in use.

As the patches, those in which an adhesive layer is laminated on a support have conventionally been popular. Normally, the adhesive layer of a patch is protected by a very thin, flexible release sheet made of a synthetic resin film, silicone-treated paper, or the like. A patch having such a configuration is attached to an affected part after peeling off the release sheet from a corner thereof groped for with a nail or the like. In many cases, the release sheet is clear and colorless and is in close contact with the adhesive layer, thus exhibiting the color of the latter. Therefore, the release sheet has been hard to peel off, thus taking a large amount of time for its peeling operation. Also, since the release sheet is soft, it has been problematic in that it cannot firmly support the patch, so that the patch may twist and get entangled, thus becoming unusable, and so forth.

Therefore, various attempts have been made in order to ameliorate the releasability of the release sheet and improve the usability of the patch. As one of them, a patch in which a release sheet is releasable from a cutout or perforation (notch) provided at a predetermined part thereof, so that an operation for peeling off the release sheet and an operation for attaching the patch can be carried out at the same time by peeling off the release sheet while applying thus exposed-adhesive layer to an affected part, is disclosed in Japanese Patent Application Laid-Open No. HEI 8-112305 and the like. Also, Japanese Patent Application Laid-Open No. HEI 10-265372 discloses a technique for improving the supportability of the patch by controlling the bending resistance of the release sheet.

However, the above-mentioned patches will still be likely to get wrinkled or entangled due to insufficient supportability of patches in their applying operation if they are provided with softness in order to prevent them from peeling. If a release sheet having a high bending resistance is used in order to enhance the supportability of patches in their applying operation, on the other hand, the release sheet will be hard to tear. Since such a vicious circle occurs, no patch has been sufficient as one which can be applied beautifully and reliably in a short time while being hard to peel off even when attached to a joint, a face, or the like for a long period of time.

### Disclosure of the Invention

In view of the above-mentioned problems of prior art, it is an object of the present invention to provide a patch which can be applied beautifully and reliably in a short time when carrying out an operation of peeling a release sheet and an operation of applying the patch to an aimed part at the same time while being hard to peel off even when attached to a joint, a face, or the like for a long period of time.

The inventors conducted diligent studies in order to achieve the above-mentioned object and, as a result, have found it hard to attain the easiness of applying the patch and resistance to peel of the patch with a favorable balance if physical properties of the release sheet are considered alone when studying the releasability of the release sheet as conventionally done, whereas the above-mentioned problems can be overcome when, in a patch comprising a laminate in which an expansible support and an adhesive layer are integrated and a release sheet having perforations formed with a predetermined interval, the load at the time of 50% elongation and 50% elongation recovery ratio of the laminate satisfy a predetermined condition, thereby accomplishing the present invention.

Namely, the present invention provides a patch comprising a laminate having an expansible support and an adhesive layer laminated on the support; and a release sheet, attached onto the adhesive layer, having perforations formed with a predetermined interval;
wherein the laminate when formed into a test piece having a longer side of 20 cm and a shorter side of 5 cm exhibits a load of 0.98 to 14.71 N/5 cm in both longer and shorter side directions at the time of 50% elongation, and a 50% elongation recovery ratio of 50 to 95% in both longer and shorter side directions.

According to the present invention, a patch comprising a laminate having an expansible support and an adhesive layer, and a release sheet having perforations with a predetermined interval is controlled such that the laminate exhibits a load of 0.98 to 14.71 N/5 cm in both longer and shorter side directions at the time of 50% elongation, and a 50% elongation recovery ratio of 50 to 95% in both longer and shorter side directions, whereby the release sheet is easily torn by simply pulling the patch leftward and rightward. Also, the patch is firmly supported when peeling the release sheet by picking a turned-up part of the release sheet, whereby the patch can be applied beautifully and reliably in a short time without making it wrinkled or entangled when carrying out an operation of peeling the release sheet and an operation of applying the patch at the same time. Controlling the laminate such that its load at the time of 50% elongation and 50% elongation recovery ratio satisfy the above-mentioned condition provides the patch (laminate) with a high skin traceability, whereby it is fully prevented from peeling even when applied to a joint, a face, or the like for a long period of time.

The load at the time of 50% elongation in accordance with the present invention refers to the value measured under the following condition in conformity to the method of measuring "elongation force (load for stretching)" defined by JIS L 1096. Namely, the load at the time of 50% elongation in accordance with the present invention refers to the force per unit width [N/5 cm] at the time when the ratio of elongation becomes 50% in each of sides in a test piece having a longer side of 20 cm and a shorter side of 5 cm pulled at a pulling rate (speed of testing rate of stressing) of 200 mm/min in the longer or shorter side direction by using a tensile tester defined by JIS Z 0237 (i.e., the longer side becomes 30 cm when pulled in the longer side direction, or the shorter side becomes 7.5 cmwhenpulled in the shorter side direction).

The 50% elongation recovery ratio in accordance with the present invention refers to the value measured under the following condition in conformity to the method of measuring "elongation recovery ratio (elastic recovery percentage of elongation)" defined by JIS L 1096. Namely, the 50% elongation recovery ratio in accordance with the present invention refers to the elongation recovery ratio [%] in the case where a step of expanding a test piece having a longer side of 20 cm and a shorter side of 5 cm at a pulling rate of 200 mm/min in the longer or shorter side direction until the elongation ratio becomes 50% and then leaving it for 1 minute, and a step of restoring it to the initial position at 200 mm/min and then leaving it for 3 minutes are repeated for 3 times, and thereafter a load on a par with the initial load is added to the test piece when further elongated at 200 mm/min.

While the patch in accordance with the present invention comprises a release sheet attached onto an adhesive layer, the load at the time of 50% elongation and 50% elongation recovery ratio are values measured when a laminate in which the expansible support and adhesive layer are integrated, i.e., the patch excluding the release sheet therefrom, is used as a test piece.

Preferably, in the present invention, the release sheet has a bending resistance of 20 to 100 mm, a tensile strength of 4.90 to 29.42 N/cm, and an elongation ratio of 250 to 750%.

Here, the bending resistance refers to the value [mm] measured by the cantilever method defined by JIS L 1085; whereas the tensile strength and elongation ratio respectively refer to the load [N/cm] and elongation ratio [%] of a strip-like test piece having a width of 10 mm with no perforations pulled at a pulling rate of 300 mm/min with a clamping width of 50 mm until it breaks according to a method in conformity to JIS Z 1702.

Preferably, in the present invention, the release sheet exhibits a tear strength of 0.147 to 1.275 N/cm when torn along the perforations. Here, the tear strength refers to the load [N/cm] in the case where a release sheet formed with perforations is used as a test piece and pulled at a pulling rate of 300 mm/min with a clamping width of 50 mm until it tears along the perforations according to a method in conformity to JIS Z 1702, thus being distinguished from the above-mentioned tensile strength.

The ratio between the length (a) of a junction between neighboring perforations and the length (b) of the perforation is preferably a:b = 1:5 to 1:30, more preferably a:b= 1:8 to 1:25. If the ratio between the length of junction and the length of perforation falls within the above-mentioned range, tearing and peeling of the release sheet at the time of use tend to become easier while fully restraining the release sheet from tearing when not in use so as not to expose the adhesive layer.

Preferably, in the patch of the present invention, the release sheet has a bending resistance of 20 to 100 mm, a tensile strength of 5 to 30 N/cm, and an elongation ratio of 250 to 750%, whereas the 180° release force between the release sheet and the adhesive layer is 0.03 to 0.4 N/40 mm.

When the release sheet has a bending resistance of 20 to 100 mm, a tensile strength of 5 to 30 N/cm, and an elongation ratio of 250 to 750%, while the 180° release force between the release sheet and the adhesive layer is 0.03 to 0.4 N/40 mm, and is provided with perforations with a predetermined interval, opposite characteristics of the releasability in the release sheet when the patch is in use and the resistance to peel of the release sheet when not in use tend to be achieved with a sufficiently favorable balance. Therefore, when carrying out an operation of peeling the release sheet and an operation of applying the patch to an affected part at the same time, the release sheet can easily be peeled off so as to apply the patch to the affected part beautifully and reliably in a short time, while the release sheet tends to be able to protect the adhesive layer without turning up when the latter is not in use.

Here, the bending resistance refers to the value [mm] measured by the cantilever method defined by JIS L 1085.

The tensile strength and elongation ratio in accordance with the present invention respectively refer to the load [N/cm] and elongation ratio [%] of a strip-like test piece having a width of 10 mm pulled at a pulling rate of 300 mm/min with a clamping width of 50 mm until it breaks according to a method in conformity to JIS Z 1702.

The 180° release force in accordance with the present invention refers to the load [N/40 mm] in the case where the release sheet is peeled from the patch under the condition of a clamping width of 40 mm and a pulling rate of 300 mm/min by using a rheometer such that the angle formed between the peeled release sheet and the patch becomes 180° according to a method in conformity to JIS C 2107.

### Brief Description of the Drawings

Fig. 1 is a perspective view showing a preferred embodiment of the patch in accordance with the present invention;
Fig. 2 is a sectional view of the patch shown in Fig. 1;
Fig. 3 is a side view for explaining an example of methods of tearing the release sheet in accordance with the present invention; and
Fig. 4 is a side view for explaining an example of methods of applying the patch of the present invention to an aimed part while peeling the release sheet.

### Best Modes for Carrying Out the Invention

In the following, preferred embodiments of the present invention will be explained in detail with reference to the drawings when necessary. In the drawings, parts identical or equivalent to each other will be referred to with numerals identical to each other.

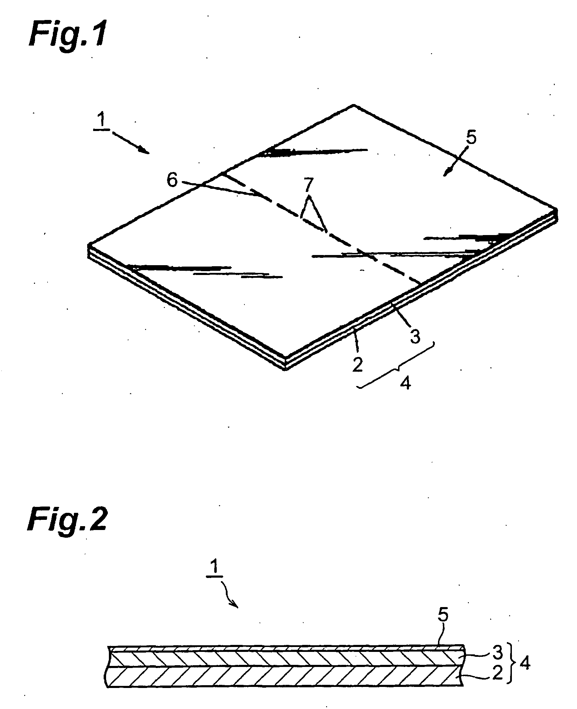

Fig. 1 is a perspective view showing a preferred embodiment of the patch in accordance with the present invention, whereas Fig. 2 is a sectional view of the main part thereof. In Figs. 1 and 2, the patch 1 comprises a laminate 4 having an expansible support 2 and an adhesive layer 3 laminated on the support 2; and a release sheet 5, attached onto the adhesive layer 3, having perforations 6 and junctions 7. In the laminate 4, the load at the time of 50% elongation is 0.98 to 14.71 N/5 cm in each of the longer and shorter side directions, whereas the 50% elongation recovery ratio is 50 to 95% in each of the longer and shorter side directions. When thus configured patch 1 of the present invention is used, the patch can be applied to an aimed part beautifully and reliably in a short time in the case where an operation of peeling the release sheet 5 and an operation of applying the patch (laminate 4) to the aimed part are carried out at the same time, while being fully prevented from peeling off in the case applied to a joint, a face, or the like for a long period of time, as will be explained later.

As mentioned above, in the laminate in accordance with the present invention, the load at the time of 50% elongation is 0.98 to 14.71 N/5 cm in both longer and shorter side directions, preferably 1.96 to 9.81 N/5 cm in the longer side direction and 0.98 to 9.81 N/5 cm in the shorter side direction. If the load at the time of 50% elongation in the laminate is less than the lower limit mentioned above, the laminate will lose so-called sturdiness (tenacity), thus failing to support the patch firmly, whereby no favorable feel of use can be obtained in the applying operation. If the load at the time of 50% elongation in the laminate exceeds the upper limit mentioned above, by contrast, the skin traceability will be insufficient, so that it will be easy to peel off upon slight movement when applied to a joint, a face, or the like.

The 50% elongation recovery ratio of the laminate in accordance with the present invention is 50 to 95% in both longer and shorter directions as mentioned above, preferably 50 to 95% in the longer side direction and 60 to 90% in the shorter side direction. If the 50% elongation recovery ratio of the laminate is less than the lower limit mentioned above, its skin traceability will become insufficient, so that it will be easy to peel off upon slight movement when applied to a joint, a face, or the like. Though the skin traceability improves as the 50% elongation recovery ratio of the laminate increases, the patch (laminate) willbe likely to get wrinkled or entangled if the upper limit is exceeded, whereby no favorable feel of use will be obtained.

The expansible support constituting the laminate in accordance with the present invention is not restricted in particular as long as the load at the time of 50% elongation and 50% elongation recovery ratio of the laminate itself satisfy the respective conditions mentioned above. Specific examples thereof include woven fabrics, knitted fabrics, nonwoven fabrics, or their laminates comprising a synthetic resin such as polyethylene terephthalate, ethylene/vinyl acetate copolymer, polyethylene polyester, nylon, polyurethane, polyamide, and rayon. Among them, polyester nonwoven fabric is preferably used, since its texture is favorable, feel of use is good, and influence upon efficacious ingredients is small. The weight per area (mass per unit area) of the support used is preferably 30 to 150 g/m², more preferably 35 to 120 g/m², particularly preferably 40 to 100 g/m². Preferably used as threads constituting the support are those having a thickness of 0.1 to 2 deniers. If the thickness of threads falls within the range mentioned above, the patch will be harder to peel off and its feel of use will tend to improve even when applied to places such as elbow, knee, and face which move greatly so that the skin expands and contracts vigorously. The support used in the present invention preferably has a thickness of 0.3 to 3 mm when it is woven fabric, knitted fabric, nonwoven fabric other than polyester, or nonwoven paper, and a thickness of 1±0.5 mm when polyester nonwoven fabric is used. If the weight per area or thickness of the support is less than its lower limit mentioned above, the patch (laminate) will be likely to get wrinkled or entangled in its applying operation, whereby a favorable feel of use will tend to be harder to attain. If the above-mentioned upper limit is exceeded, by contrast, the flexibility of the patch (laminate) will become insufficient, whereby uncomfortable feeling such as a feel of being drawn will tend to occur at the time of applying the patch.

The load at the time of 50% elongation in the expansible support used in the present invention is preferably 0.98 to 14.71 N/5 cm in both longer and shorter side directions, more preferably 1.96 to 9.81N/5 cm in the longer side direction and 0.98 to 9.81 N/5 cm in the shorter side direction. If the load at the time of 50% elongation in the support is less than the lower limit mentioned above, the laminate may lose so-called sturdiness, thus failing to support the patch firmly, whereby a favorable feel of use will tend to be harder to obtain in applying operations. If the load at the time of 50% elongation in the support exceeds the upper limit mentioned above, by contrast, its skin traceability may become insufficient, so that it will tend to be easier to peel off upon slight movement when applied to a joint, a face, or the like.

The 50% elongation recovery ratio of the expansible support used in the present invention is preferably 50 to 95% in both longer and shorter side directions, more preferably 50 to 95% in the longer side direction and 60 to 90% in the shorter side direction. If the 50% elongation recovery ratio of the laminate is less than the lower limit mentioned above, its skin traceability may become insufficient, so that it will tend to be easier to peel off upon slight movement when applied to a joint, a face, or the like. Though the skin traceability improves as the 50% elongation recovery ratio of the laminate increases, the patch (laminate) will be likely to get wrinkled or entangled in applying operations if the upper limit is exceeded, whereby a favorable feel of use will tend to be harder to attain.

Though the adhesive layer constituting the laminate is not restricted in particular as long as the load at the time of 50% elongation and 50% elongation recovery ratio of the laminate itself satisfy the conditions mentioned above, one having an adhesive force which can fix a medicine onto a skin surface for a long time at room temperature is preferable. For example, when the patch of the present invention is employed as a poultice, it is mainly composed of water, a thickener, a wetting agent, a filler, and the like, while other ingredients such as crosslinking agent, polymerizer, solubilizing agent, absorption accelerator, efficacy adjuvant, stabilizer, antioxidant, emulsifier, refrigerant, and medicine are added thereto if necessary.

Preferably, the thickener used in the present invention can stably keep a moisture of 10 to 85% by mass (preferably 25 to 65% by mass) and has a water retentivity. Preferably employable as specific examples of such a thickener are water-soluble polymers such as natural polymers including those of vegetable type such as guar gum, locust bean gum, carrageenan, alginicacid, sodium alginate, agar, gumarabic, tragacanth gum, karaya gum, pectin, and starch, microorganic type such as xanthan gum and acacia gum, and animal type such as gelatin and collagen; semi-synthetic polymers including those of cellulose type such as methyl cellulose, ethyl cellulose, hydroxyethyl cellulose, caryboxymethyl cellulose, and sodium carboxymethyl cellulose, and starch type such as soluble starch, carboxymethyl starch, and dialdehyde starch; and synthetic polymers including those of vinyl type such as polyvinyl alcohol, polyvinyl pyrrolidone, and polyvinyl methacrylate, acrylic type such as polyacrylic acid and sodium polyacrylate, and other synthetic polymers such as polyethylene oxide and methyl vinyl ether/maleic anhydride copolymer. In particular, as such a thickener, sodium polyacrylate having a high gel strength and excellent water retentivity is preferably used, and sodium polyacrylate having an average degree of polymerization of 20000 to 70000 is further preferably used. A sufficient gel strength tends to be harder to attain as the average degree of polymerization decreases from 20000, whereas the thickening effect tends to become so strong that operability may lower as the average degree of polymerization increases from 70000, both of which are unfavorable. When two or more kinds of the water-soluble polymers are used, a polymer complex can be formed with a polymer having stronger ions of sodium polyacrylate, for example, whereby an elastic gel having a higher gel strength can be obtained. The compounding amount of the thickener used in the present invention is preferably 1 to 50% by mass, more preferably 3 to 30% by mass, based on the total amount of ingredients contained in the adhesive layer, whereby moisture retention, stability, skin adhesion, medicine evaporation prevention, percutaneous absorption, clogginess and fluidization prevention, pain prevention upon peeling, adhesive layer leftover prevention, and the like can be improved.

Examples of the wetting agent used in the present invention include polyhydric alcohols such as glycerin, propylene glycol, and sorbitol. The compounding amount of the wetting agent is preferably 3 to 60% by mass, more preferably 10 to 45% by mass, based on the total amount of ingredients in the adhesive layer, where by moisture retention, coolability, medicine evaporation prevention, percutaneous absorption, and the like can be improved. The compounding amount of the wetting agent is preferably 3 to 60% by mass, more preferably 10 to 45% by mass, based on the total amount of ingredients in the adhesive layer, whereby moisture retention, coolability, medicine evaporation prevention, percutaneous absorption, and the like can be improved.

Examples of the filler used in the present invention include kaolin, zinc oxide, talc, titanium, bentonite, aluminum silicate, titanium oxide, zinc oxide, aluminum metasilicate, calcium sulfate, calcium phosphate, and the like. The compounding amount of the filler is preferably 0 to 15% by mass, more preferably 0.01 to 10% by mass, based on the total amount of ingredients in the adhesive layer.

Also, propylene carbonate, crotamiton, L-menthol, mentha oil, limonene, diisopropyl adipate, and the like as a solubilizing agent or absorption accelerator; and methyl salicylate, glycol salicylate, L-menthol, thymol, mentha oil, limonene, nonylic acid vanillylamide, capsicum extract, and the like as an efficacy adjuvant may be added to the adhesive layer (ointment) in accordance with the present invention. Further, when necessary, stabilizers, antioxidants, emulsifiers, refrigerants, and the like may be added to the adhesive layer in accordance with the present invention.

Further, when necessary, crosslinking agents, polymerizers, and the like may be added to the adhesive layer in accordance with the present invention, so as to strengthen the adhesive layer and impart a water retentivity thereto. Such crosslinking agents and polymerizers can appropriately be chosen according to the kind of thickeners and the like. For example, in the case where polyacrylic acid or polyacrylate is employed as a thickener, compounds having at least two pieces of epoxy group in a molecule; inorganic acid salts such as hydrochlorides, sulfates, phosphates, and carbonates of Ca, Mg, Al, and the like; organic acid salts such as citrates, tartrates, gluconates, and stearates; oxides such as zinc oxide and silicic anhydride; and polyvalent metal compounds such as hydroxides like aluminum hydroxide and magnesium hydroxide are preferably used. In the case where polyvinyl alcohol is employed as a thickener, adipic acid, thioglycolic acid, epoxy compounds (epichlorohydrin), aldehydes, N-methylol compounds, complexes such as compounds of Al, Ti, Zr, Sn, V, Cu, B, and Cr, and the like are preferably used. When polypyrrolidone is employed as a thickener, methyl vinyl ester/maleic anhydride copolymer, polyacid compounds, alkali metal salts (polyacrylic acid, tannic acid, and their derivatives), and the like are preferably used. When polyethylene oxide is employed as a thickener, peroxide, polysulfone azide, and the like are preferably used. When methyl vinyl ether/maleic anhydride copolymer is employed as a thickener, polyfunctional hydroxy compounds, polyamines, iodine, gelatin, polyvinylpyrrolidone, iron, mercury, lead salts, and the like are preferably used. When gelatin is employed as a thickener, aldehydes such as formaldehyde, glutaraldehyde, and dialdehyde starch; diepoxides such as glyoxal and butadiene oxide; diketones such as divinyl ketone; and diisocyanates are preferably used.

When thus configured patch is caused to hold a medicine, it will be sufficient if the medicine is compounded in the adhesive layer. Examples of the medicine used in the patch of the present invention include general anesthetics, hypnotics, analgesics, antipyretic/antiphlogistic analgesics, steroid hormone drugs, analeptics/stimulants, psychiatric/neurological drugs, local anesthetics, skeletal muscle relaxants, drugs for autonomic nerves, antiallergic agents, antihistamine agents, cardiotonics, drugs for arrhythmia, diuretics, hypotensive agents, vasoconstrictors, vasodilators, calcium antagonists, antibacterial/bactericidal agents, drugs for parasitic dermatosis, skin softeners, antibiotics, antidotes, cough remedies, antipruritics, sleeping drugs, psychiatric activators, drugs for asthma, hormone secretion accelerators, antiulcer agents, anticancer drugs, vitamin compounds, and the like. More specific examples of such a medicine include steroid type antiinflammatory medicines (e.g., prednisolone, dexamethasone, hydrocortisone, betamethasone, fluocinonide, fluocinolone acetonide, prednisolone acetate valerate, dexamethasone dipropionate, diflucortolone valerate, difluprednate, betamethasone valerate, hydrocortisone butyrate, clobetasone butyrate, betamethasone butyrate, clobetasone propionate, dexamethasone succinate, prednisolone-21-(2E, 6E) farnesylate, hydrocortisone valerate, diflorasone diacetate, dexamethasone propionate, betamethasone dipropionate, amcinonide, dexamethasone valerate, halcinonide, budesonide, alclometasone dipropionate, and the like), nonsteroid type antiinflammatory medicines (e.g., methyl salicylate, glycol salicylate, 1-menthol, capsicum extract, nonylic acid vanillylamide, mentha oil, diclofenac, ibuprofen, indomethacin, ketoprofen, loxoprofen, sulindac, tolmetin, lobenzarit, penicillamine, fenbufen, flurbiprofen, suprofen, felbinac, ketorolac, oxaprozin, etodolac, zaltoprofen, piroxicam, pentazocine, buprenorphine hydrochloride, butorphanol tartrate, and the like), analgesics (e.g., fentanyl citrate, morphine hydrochloride, butorphanol tartrate, pentazocine, and the like), hormone drugs (e.g., estradiol, norethisterone, and the like), local anesthetics (e.g., lidocaine, dibucaine hydrochloride, and the like), antihistamine agents (e.g., chlorpheniramine maleate and the like), antiallergic agents (e.g., mequitazine, ketotifen fumarate, and the like), skeletal muscle relaxants (e.g., eperisone, dantrolene, and the like), psychiatric/neurological drugs, antiemetics (e.g., granisetron hydrochloride, azasetron hydrochloride, and the like), calcium antagonists (e.g., nifedipine, nicardipine, and the like), coronary vasodilators (e.g., nitroglycerin, isosorbide dinitrate, and the like), antifungals (e.g., clotrimazole and the like), antineoplastics (e.g., bleomycin, 5-fluorouracil, and the like), hair growth stimulants (e.g., minoxidil and the like), refrigerants (e.g., mentha oil, menthol, and the like), whiteners (e.g., kojic acid and the like), vitamines, juices and the like, and hormones and the like. One kind of these medicines may be used alone, or two or more kinds thereof may be used as a mixture. Also, they may be used in the form of derivatives such as ester, acetal, and amide, or medically tolerable inorganic and organic salts. The compounding amount of medicines used in the present invention is preferably 0.001 to 60% by weight based on the total amount of ingredients contained in the adhesive layer. Further, the amount of medicine used in the patch of the present invention is appropriately selected such that a predetermined effective amount thereof can be applied to an affected part. The patch can be compounded with whitening ingredients, skincare ingredients, and the like in addition to the above-mentioned medicines.

When the patch of the present invention is employed in a tape preparation, it may be mainly composed of a medicine, an adhesive, a tackifier, aplasticizer, and the like, whereas the antioxidants, UV-absorbers, fillers, refrigerants, and the like exemplified in the above-mentioned explanation of poultice can be compounded therewith as required.

Examples of the adhesive used in the tape preparation in accordance with the present invention include styrene/isoprene/styrene block copolymer, polyisoprene, polyisobutylene, acrylic copolymers (copolymers of at least one of butyl acrylate, 2-ethylhexyl acrylate, methacrylate, and hydroxyethyl acrylate, and the like) , and the like. One kind of them may be used alone, or two or more kinds thereof maybe used as a mixture. The compounding amount of adhesives mentioned above except for acrylic copolymers is preferably 10 to 50% by mass based on the total amount of ingredients contained in the adhesive layer. If the compounding amount of adhesive is less than the lower limit mentioned above, the cohesive force and shape retentivity of the adhesive layer will tend to become insufficient. If the compounding amount of the adhesive exceeds the upper limit mentioned above, by contrast, the cohesive force of the adhesive layer will become too large, so that the adhesive force may lower or the adhesive layer may become uneven, whereby peeling will be likely to occur when the adhesive layer is applied to a joint, a face, or the like for a long period of time. On the other hand, the compounding amount of acrylic copolymer is preferably 5 to 95% by mass based on the total amount of ingredients contained in the adhesive layer. If the compounding amount of acrylic copolymer is less than the lower limit mentioned above, the adhesive force will become insufficient, so that peeling will be likely to occur when the adhesive layer is applied to a joint, a face, or the like for a long period of time. If the compounding amount of acrylic copolymer exceeds 95% by mass, by contrast, there will be a tendency of skins being irritated or pains occurring when peeling the adhesive layer from the applied part.

Specific examples of the tackifier used in the tape preparation in accordance with the present invention include rosin esters, hydrogenated rosin esters, rosin maleate, aliphatic saturated hydrocarbon resins, terpene phenol, and the like. One kind thereof may be used alone, or two or more kinds thereof may be used as a mixture. The compounding amount of tackifier is preferably 5 to 50% by mass based on the total amount of ingredients contained in the adhesive layer. If the compounding amount of tackifier is less than the lower limit mentioned above, the adhesive force will become insufficient, so that peeling will be likely to occur when the adhesive layer is applied to a joint, a face, or the like for a long period of time. If the compounding amount of tackifier exceeds the upper limit mentioned above, by contrast, the percutaneous absorption of medicine, the shape retentivity of adhesive layer, and the like will lower, whereas there will be a tendency of pains and clogginess occurring upon peeling the adhesive from the applied part and skins being irritated.

Examples of the plasticizer used in the tape preparation in accordance with the present invention include liquid paraffin, squalane, squalene, vegetable oils, liquid polybutene, and the like. Preferably, the compounding amount of plasticizer is 20 to 60% by mass based on the total amount of ingredients contained in the adhesive layer. If the compounding amount of plasticizer is less than the lower limit mentioned above, the percutaneous absorption of the medicine and the adhesive force of the adhesive layer will tend to lower. If the compounding amount of plasticizer exceeds the upper limit mentioned above, by contrast, the cohesive force and shape retentivity of the adhesive layer will lower, and there will be a tendency of pains and clogginess occurring upon peeling the adhesive from the applied part and skins being irritated.

A preferred example of the adhesive layer in the case where the patch of the present invention is employed in a poultice is one comprising the following composition:
thickener: 20 to 50% by mass (more preferably 10 to 15% by mass)
wetting agent: 5 to 40% by mass
filler: 0 to 20% by mass
water: 10 to 80% by mass
solubilizing agent: 0 to 8% by mass
medicine: 0 to 5% by mass (preferably 0.5 to 5% by mass)

If the ingredients of adhesive layer fall within their ranges mentioned above, characteristics such as favorable skin contact, high skin absorption of effective ingredients, large water capacity, no fluidization at ambient temperature or in the vicinity thereof, no pains at the time of peeling the patch, and no smudges or clogginess remaining after peeling the patch tends to be able to be achieved with a very high level.

Preferably used as a base when the patch of the present invention is employed in a plaster or the like is one having an acrylic copolymer, an A-B-A type block copolymer, an alicyclic petroleum resin, and a softener. Further compounded are thermoplastic elasticizers such as A-B type block copolymer, polybutene rubber, butyl rubber, silicone rubber, natural rubber, styrene/butadiene copolymer, NBR polyisobutylene, poly(alkyl acrylate), and synthetic isoprene; tackifiers such as terpene resins, petroleum resins, rosin, hydrogenated rosin, and rosin/hydrogenated rosin ester; animal and vegetable oils such as liquid paraffin, olive oil, soybean oil, beef tallow, and lard; bonding force/retentivity regulator such as polybutene, liquid polyisobutylene, lower isoprene, and wax; and fillers such as titanium oxide, zincoxide, aluminummetasilicate, calcium sulfate, and calcium phosphate. Preferably used as the A-B-A type block copolymer or A-B type block copolymer is a block copolymer formed by a monovinyl-substituted aromatic compound A and a conjugated diolefin copolymer B, whereas its compounding amount is 10 to 40% by mass, preferably 15 to 30% by mass, in the adhesive composition. If the compounding ratio of the block copolymer falls within the above-mentioned range, the compatibility, anchoring force, and adhesive force of efficacious ingredients will improve, while the cohesive force caused by oily ingredients can be ameliorated. As the compounding ratio shifts from such a range, these effects tend to lower.

Preferably, the thickness (coating thickness) of the adhesive layer laminated on the support in the patch of the present invention is 0.1 mm to 3 mm. The releasability of the medicine contained in the ointment will tend to deteriorate if the thickness of the adhesive layer exceeds 3 mm, whereas the adhesion to skins will tend to deteriorate and cause peeling if the thickness is less than 0.1 mm.

Preferably, the thickness including the support and adhesive layer in the patch of the present invention is 0.5 mm to 5 mm. If the thickness including the support and adhesive layer exceeds 5 mm, edges of the patch will be likely to catch clothes and the like during the patch is applied and tend to peel off. If the thickness is less than 0.5 mm, by contrast, the patch will tend to lose its supportability and get entangled or wrinkled, thus becoming harder to apply beautifully and easily in a short time.

Preferably, in the patch of the present invention, its corners are rounded. Once a patch is partly turned up and peeled after the release sheet is peeled off, horny cell layers of skins and dust are likely to attach thereto, thus making it difficult to reapply. In particular, when a poultice has a rectangular form, its corners are likely to catch clothes and the like, turn up at first, and peel off. Therefore, the poultice is preferably formed with rounded corners so as to make them harder to catch clothes and the like, whereby peeling tends to be more effectively prevented from occurring.

When corners in the patch of the present invention are to be rounded, thus rounded corners preferably have a radius of curvature (R) of 5 mm to 20 mm. If the radius (R) is less than 5 mm, lowering of generating-probability of peeling during the patch is applied may not fully be achieved. If the radius (R) exceeds 20 mm, by contrast, the phenomenon of peeling from corners can be prevented from occurring, but wastes in manufacturing will be so large that the manufacturing cost will rise, thus tending to be less profitable.

Conventional patches have been disadvantageous in that the release sheet becomes harder to peel while the release prevention effect can be obtained by rounding their corners . By contrast, the patch of the present invention satisfies opposite characteristics of the releasability of the release sheet when in use and the resistance to peel of the release sheet when not in use with a sufficiently favorable balance, whereby the release sheet is easy to tear and peel from its perforations. Also, the release prevention inherent in the patch of the present invention and the difficulty in peeling due to the rounded corners can synergistically improve the release prevention effect.

Specific examples of the release sheet used in the present invention include plastic films such as oriented or non-oriented polypropylene, polyethylene terephthalate, polybutylene terephthalate, polyethylene, polyvinyl chloride, polyester, polyvinylidene chloride, polyurethane, and polystyrene; silicone-treated paper such as silicone-treated synthetic resin, synthetic paper, and synthetic fiber; aluminum foil; and laminate-processed paper in which polyethylene or the like is laminated on kraft paper, while being left uncolored or colored, are preferably used. The thickness of release sheet is normally 10 µm to 100 µm, preferably 12 µm to 70 µm, more preferably 15 µm to 50 µm. If the thickness of release sheet is less than the lower limit mentioned above, the release sheet will be likely to get entangled with the adhesive layer, while the release sheet will be harder to pick, whereby the patch will tend to be harder to apply beautifully and reliably in a short time. In addition, the release sheet will be easier to tear or get wrinkled in the process of making the patch, and so forth, whereby operability will tend to lower. Though the release sheet becomes easier to grip as its thickness increases, it will be harder to tear and will lower the feel of use in applying operations if the upper limit is exceeded. In addition, the original sheet for the release sheet will be harder to cut in the process of making the patch, and so forth, whereby operability will tend to lower.

As shown in Figs. 1 and 2, the release sheet used in the present invention has perforations 6 formed with predetermined intervals, and junctions 7 each formed between a pair of neighboring perforations 6, 6. Though positions formed with perforations are not restricted in particular, they are usually formed at substantially the center part of the release sheet or its surroundings. The form of perforations in accordance with the present invention may be linear, S-shaped, wavy, saw-tooth, and so forth. Among them, S-shaped, wavy, or saw-tooth perforations are particularly preferable, since protrusions formed with predetermined intervals can be picked up and pulled, which improves the easiness of peeling the release sheet in applying operations.

The ratio between the length of junction (a) formed between neighboring perforations and the length of perforation (b) is preferably a:b = 1:5 to 1:30, more preferably a:b = 1:8 to 1:20, further preferably a:b = 1:8 to 1:15. If the length of perforation is less than 5 times the length of junction, the release sheet will be harder to tear, whereby the feel of use in applying operations will tend to lower. If the length of perforation exceeds 30 times the length of junction, the release sheet will be likely to tear even when not in use, thus yielding a tendency of the release sheet turning up from the torn part, so that the patch will get entangled, and efficacious ingredients evaporating from the torn part, thereby lowering the efficacious effect.

The bending resistance of the release sheet in accordance with the present invention is preferably 20 to 100 mm, more preferably 30 to 70 mm. If the bending resistance of the release sheet is less than the lower limit mentioned above, the supportability of release sheet will lower, whereby the patch will be likely to get wrinkled or entangled in applying operations. If the bending resistance of release sheet exceeds the upper limit mentioned above, by contrast, the softness of release sheet will lower, so that the patch will be harder to apply beautifully and reliably in a short time.

The tensile strength of the release sheet in accordance with the present invention is preferably 5 to 30 N/cm, more preferably 10 to 25 N/cm. Though the release sheet becomes easier to tear as its tensile strength decreases, the release sheet will be torn by a minute external force exerted at the time of making the patch or taking out the patch if the lower limit mentioned above is not satisfied, whereby phenomena such as entangling of the patch and evaporation of efficacious ingredients from the torn part will be likely to occur. If the tensile strength of release sheet exceeds the upper limit mentioned above, by contrast, a strong force will be needed for tearing the release sheet, whereby the feel of use and working efficiency at the time of applying the patch will tend to lower.

The elongation ratio of the release sheet in accordance with the present invention is preferably 250 to 750%, more preferably 300 to 600%. Though the release sheet becomes easier to tear as the elongation ratio of the release sheet decreases, the release sheet will be torn upon a minute external force exerted at the time of making the patch or taking out the patch from its package bag if the elongation ratio is less than 250%, whereby phenomena such as entangling of the patch and evaporation of efficacious ingredients from the torn part will be likely to occur. If the elongation ratio of release sheet exceeds 750%, by contrast, the release sheet will be less likely to tear although it is fully extended, whereby the feel of use and working efficiency at the time of applying the patch will tend to lower. The above-mentioned plastic films are favorably used as a release sheet whose bending resistance, tensile strength, and elongation ratio satisfy the above-mentioned conditions.

The tear strength of the release sheet in accordance with the present invention is preferably 0.147 to 1.275 N/cm, more preferably 0.196 to 0.883 N/cm, further preferably 0.245 to 0.686 N/cm. If the tensile strength at the time of tearing the release sheet at perforations is less than the lower limit mentioned above, the release sheet will be easier to tear in the process of making the patch, whereby continuous operations will be harder to carry out. Also, when accommodating the patch into a package bag, the release sheet will be easier to tear upon a minute external force, so that efficacious ingredients will evaporate from the torn part, and so forth, whereby the quality will tend to lower. If the tensile strength at the time of tearing the release sheet at perforations exceeds the upper limit mentioned above, the tearing property of release sheet will lower, whereby a favorable feel of use will be harder to attain in applying operations.

Though not shown in Figs. 1 and 2, the whole surface or a part of the surface of the release sheet is preferably provided with an embossed part in the present invention. When an embossed release sheet is used, hands are unlikely to slip in operations of tearing and releasing the release sheet, whereby the working efficiency tends to become higher. The form of embossed part may specifically be grids, circles, polygons, stars, and the like, though not restricted in particular as long as it is hard to slip but easy to pick with a hand. When a part of the release sheet surface is to be embossed, its position is not restricted in particular . Embossing a part which can easily be picked with a hand when tearing the release sheet, i.e., a part having a width of 10 to 20 mm from each end part of perforations, makes it possible to tear the release sheet reliably without slipping hands. Since the release sheet turns up from perforations when torn as will be explained later, the release sheet turned up after tearing can be peeled off more reliably if the embossed part is provided near the perforations. Here, for clarifying a method of tearing the release sheet, indicators printed with figures such as arrows, letters, symbols, and the like can be provided on both sides of the perforations in the release sheet surface.

In the present invention, the 180° release force between the release sheet and the adhesive layer is preferably 0.03 to 0.4 N/40 mm, more preferably 0.05 to 0.3 N/40 mm. If the 180° release force between the release sheet and the adhesive layer is less than the lower limit mentioned above, the release sheet will be likely to peel off at the time of making or storing the patch, thus tending to cause entangling of the patch or evaporation of efficacious ingredients from the peeled part. If the 180° release force between the release sheet and the adhesive layer exceeds the upper limit mentioned above, by contrast, the release sheet will be harder to peel from the patch, whereby the feel of use and working efficiency will lower when applying the patch, and the patch will be easier to get entangled or wrinkled, thereby becoming harder to apply beautifully.

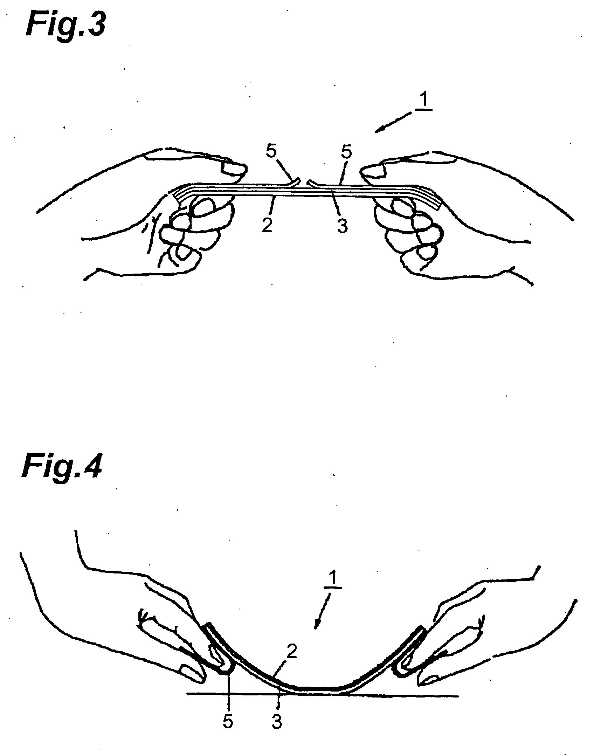

An example of methods of using thus configured patch of the present invention will be explained in the following. Fig. 3 is an explanatory view showing an operation of tearing the release sheet of the patch shown in Fig. 1, whereas Fig. 4 is an explanatory view showing an operation of applying the patch (laminate) while peeling the release sheet. First, as shown in Fig. 3, both ends of the patch 1 are picked, and the release sheet 5 is pulled together with the support 2, whereby the release sheet 5 can be torn along the perforations 6 rightward and leftward. Thus torn release sheet turns up due to the elasticity of release sheet from the torn part, thereby exposing the adhesive layer 3. Subsequently, as shown in Fig. 4, thus exposed adhesive layer 3 is applied to an aimed part, whereas thus turned-up strips of the release sheet 5 are respectively pulled rightward and leftward so as to be peeled off, whereby an operation of peeling the release sheet 5 and an operation of applying the patch 1 to the affected part are carried out at the same time. Thus, the patch of the present invention can be applied to the affected part beautifully and easily in a short time without being entangled or wrinkled.

### [Examples]

In the following, the present invention will be explained more specifically with reference to Examples and Comparative Examples, which will not restrict the present invention.

### Example 1

A coating liquid was prepared by compounding 10% by mass of sodium polyacrylate, 16% by mass of glycerin, 10% by mass of kaolin, 1% by mass of L-menthol, 3% by mass of ketoprofen, and 60% by mass of purified water and mixing them uniformly. Thus obtained coating liquid was spread on an expansible polyester nonwoven fabric support (load at the time of 50% elongation: 5.5 N/cm; 50% elongation recovery ratio: 92%) so as to attain a coating thickness of 1300 µm, thereby forming an adhesive layer, thus yielding an aimed laminate. The load at the time of 50% elongation in thus obtained laminate was 4.90 N/5 cm in the longer side direction and 0.98 N/5 cm in the shorter side direction, whereas its 50% elongation recovery ratio was 70% in the longer side direction and 50% in the shorter side direction.

Subsequently, a non-oriented polypropylene release sheet (bending resistance: 20 mm; tensile strength: 10 N/cm; elongation ratio: 280%; tear strength at perforations: 0.49 N/cm) having perforations formed at substantially the center part thereof such that the ratio between the length of junction (a) and the length of perforation (b) became a:b = 1:15 was attached to the whole surface of the adhesive layer of the above-mentioned laminate, thereby preparing a poultice.

### Example 2

A coating liquid was prepared by compounding 23% by mass of sodium polyacrylate, 13% by mass of glycerin, 10% by mass of kaolin, 1% by mass of L-menthol, 1% by mass of indomethacin, and 52% by mass of purified water and mixing them uniformly. Thus obtained coating liquid was spread on an expansible polyester nonwoven fabric support (load at the time of 50% elongation: 5.5 N/cm; 50% elongation recovery ratio: 92%) so as to attain a coating thickness of 1100 µm, thereby forming an adhesive layer, thus yielding an aimed laminate. The load at the time of 50% elongation in thus obtained laminate was 5.88 N/5 cm in the longer side direction and 1.96 N/5 cm in the shorter side direction, whereas its 50% elongation recovery ratio was 80% in the longer side direction and 60% in the shorter side direction.

Subsequently, a polyethylene terephthalate release sheet (bending resistance: 80mm; tensile strength: 26 N/cm; elongation ratio: 600%; tear strength at perforations: 0.69 N/cm) having perforations formed at substantially the center part thereof such that the ratio between the length of j unction (a) and the length of perforation (b) became a:b = 1:7 was attached to the whole surface of the adhesive layer of the above-mentioned laminate, thereby preparing a poultice.

### Example 3

A uniform dispersion was prepared by mixing 48.8% by mass of purified water, 9.5% by mass of kaolin, 0.3% by mass of titanium oxide, 0.6% by mass of sorbitan sesquioleate (HLB value = 4.3), 0.5% by mass of synthetic aluminum silicate, and 0.3% by mass of tartaric acid. To this dispersion, a dispersion prepared beforehand from 9% by mass of sodium polyacrylate, 24% by mass of glycerin, and 3% by mass of sodium carboxymethyl cellulose was added, so as to prepare a uniformly kneaded product. A solution of 1% by mass of felbinac dissolved in 3% by mass of crotamiton was added to and mixed with the kneaded product, so as to yield a coating liquid. Thus obtained coating liquid was spread on an expansible polyester nonwoven fabric support (load at the time of 50% elongation: 5.5 N/cm; 50% elongation recovery ratio: 92%) so as to attain a coating thickness of 800 µm, thereby forming an adhesive layer, thus yielding an aimed laminate. The load at the time of 50% elongation in thus obtained laminate was 6.37 N/5 cm in the longer side direction and 2.45 N/5 cm in the shorter side direction, whereas its 50% elongation recovery ratio was 70% in the longer side direction and 55% in the shorter side direction.

Subsequently, an oriented polypropylene release sheet (bending resistance: 40 mm; tensile strength: 22 N/cm; elongation ratio: 350%; tear strength at perforations: 0.3 N/cm) having perforations formed at substantially the center part thereof such that the ratio between the length of junction (a) and the length of perforation (b) became a:b = 1:20 was attached to the whole surface of the adhesive layer of the above-mentioned laminate, thereby preparing a poultice.

### Example 4

A coating liquid was prepared by uniformly mixing 21% by mass of styrene/isoprene/styrene block copolymer, 5% by mass of polyisobutylene, 14% by mass of hydrogenated rosin ester, 55% by mass of liquid paraffin, 1% by mass of dibutylhydroxytoluene, 1% by mass of synthetic aluminum silicate, and 3% bymass of ketoprofen. Thus obtained coating liquid was spread on an expansible polyester nonwoven fabric support (load at the time of 50% elongation: 5.5 N/cm; 50% elongation recovery ratio: 92%) so as to attain a coating thickness of 150 µm, thereby forming an adhesive layer, thus yielding an aimed laminate. The load at the time of 50% elongation in thus obtained laminate was 4.90 N/5 cm in the longer side direction and 1.47 N/5 cm in the shorter side direction, whereas its 50% elongation recovery ratio was 90% in the longer side direction and 75% in the shorter side direction.

Subsequently, a polyethylene terephthalate release sheet (bending resistance: 80 mm; tensile strength: 26 N/cm; elongation ratio: 600%; tear strength at perforations: 0.29 N/cm) having perforations formed at substantially the center part thereof such that the ratio between the length of junction (a) and the length of perforation (b) became a:b = 1:25 was attached to the whole surface of the adhesive layer of the above-mentioned laminate, thereby preparing a tape preparation.

### Comparative Example 1

A poultice was prepared in the same manner as Example 1 except that a nonexpansible polyester support (product name: Tetoron film manufactured by Teijin) was used as a support.

### Comparative Example 2

A tape preparation was prepared in the same manner as Example 4 except that a nonexpansible polyester support (product name: Tetoron film manufactured by Teijin) was used as a support.

Thus obtained patches of Examples 1 to 4 and Comparative Examples 1 and 2 were subjected to the following tests.

### Test 1 (Tearing Test of Release Sheet)

As a tester, each of 10 healthy adult men and women evaluated the patches of Examples 1 to 4 and Comparative Examples 1 and 2 in terms of easiness of tearing when tearing their release sheets by pulling them leftward and rightward according to the following criteria:
5: very easy to tear
4: easy to tear
3: relatively easy to tear
2: hard to tear
1: very hard to tear
Table 1 shows average values of thus obtained points.

### Test 2 (Applying Test of Patch)

As a tester, each of 10 healthy adult men and women evaluated the patches of Examples 1 to 4 and Comparative Examples 1 and 2 in terms of feel of use when applying them (laminates) to knees while peeling their release sheets after tearing the release sheets by pulling them leftward and rightward according to the following criteria:
5: very easy to apply
4: easy to apply
3: relatively easy to apply
2: hard to apply
1: very hard to apply
Table 1 shows average values of thus obtained points.

### Test 3 (Peel Prevention Test of Patch)

The patches (laminates) of Examples 1 to 4 and Comparative Examples 1 and 2 applied to the knees of testers in the above-mentioned Test 2 were evaluated in terms of the resistance to peel after the lapse of 1 day according to the following criteria:
3: no peeling occurred
2: peeling occurred in less than 20% of the area to which the patch (laminate) was applied
1: peeling occurred in at least 20% of the area to which the patch (laminate) was applied
Table 1 shows average values of thus obtained points.

**TABLE 1**

| | Test1 (Easiness of tearing release sheet) | Test2 (Easiness of applying) | Test3 (Resistance to peel) | Overall evaluation evaluation |
|---|---|---|---|---|
| Example1 | 5 | 5 | 3 | ⓞ |
| Example2 | 5 | 5 | 3 | ⓞ |
| Example3 | 4 | 5 | 3 | ○ |
| Example4 | 4 | 5 | 3 | ○ |
| Comparative example1 | 1 | 1 | 1 | × |
| Comparative example2 | 1 | 1 | 1 | × |

As shown in Table 1, the patches of Examples 1 to 4 exhibited excellent results in all of the easiness of tearing the release sheets, easiness of applying the patches, and resistance to peel of the patches.

By contrast, the patches of Comparative Examples 1 and 2 required a large force to tear their release sheets, whereas the release sheets were hard to turn up when simply tearing them. Such a patch was found to be disadvantageous in that, when applying it, the release sheet had to be picked while groping for the torn part with fingers, whereby the patch was hard to apply, and fingertips were likely to be smudged. In addition, they were likely to peel off upon slight movement of parts to which they were applied.

### Example 5

According to the following procedure, a patch shown in Fig. 1 was prepared.

As a support, an expansible polyester nonwoven fabric (load at the time of 50% elongation: 4.90 N/5 cm; 50% elongation recovery ratio: 75%; weight per area: 100 g/m²; thread thickness: 1 denier) was used. A thickener made of 5% by mass of sodium polyacrylate, 2% by mass of polyvinyl alcohol, 2% by mass of kaolin, 10% by mass of glycerin, and 0.5% by mass of aluminum hydroxide; 0.5% by mass of ketoprofen as a medicine; and 80% by mass of water were compounded and mixed, and the resulting mixture was used as an ointment. The ointment was spread on the support so as to attain a coating thickness of 1000 µm, thereby preparing an adhesive layer. The load at the time of 50% elongation in thus obtained laminate was 5.20 N/5 cm in the longer side direction and 1.76 N/5 cm in the shorter side direction, whereas its 50% elongation recovery ratio was 65% in the longer side direction and 70% in the shorter side direction.

Subsequently, non-oriented polypropylene (thickness: 30 µm; bending resistance: 20 mm; tensile strength: 5 N/cm; elongation ratio: 280%) was used as a release sheet. This release sheet was attached to the whole surface of the above-mentioned adhesive layer, and linear perforations were formed at substantially the center part of the adhesive layer such that the ratio between the length of junction (a) and the length of perforation (b) became a:b = 1:8, whereby an aimed patch (longer side: 14 cm; shorter side: 10 cm) was obtained. The 180° release force between the release sheet and adhesive layer in thus obtained patch was 0.15 N/40 mm.

### Example 6

A patch was prepared in the same manner as Example 5 except that a release sheet made of non-oriented polypropylene (thickness: 50 µm; bending resistance: 50 mm; tensile strength: 22 N/cm; elongation ratio: 730%) was used in place of the release sheet used in Example 5. The 180° release force between the release sheet and adhesive layer in thus obtained patch was 0.05 N/40 mm.

### Example 7

A patch was prepared in the same manner as Example 5 except that a release sheet made of polyethylene (PE) (thickness: 40 µm; bending resistance: 60 mm; tensile strength: 14.7 N/cm; elongation ratio: 410%) was used in place of the release sheet used in Example 5. The 180 ° release force between the release sheet and adhesive layer in thus obtained patch was 0.3 N/40 mm.

### Example 8

A patch was prepared in the same manner as Example 5 except that a release sheet made of polyethylene (thickness : 50 µm; bending resistance: 80 mm; tensile strength: 26 N/cm; elongation ratio: 600%) was used in place of the release sheet used in Example 5. The 180° release force between the release sheet and adhesive layer in thus obtained patch was 0.4 N/40 mm.

### Example 9

A patch was prepared in the same manner as Example 5 except that a release sheet made of non-oriented polypropylene (thickness: 100 µm; bending resistance: 100 mm; tensile strength: 30 N/cm; elongation ratio: 310%) was used in place of the release sheet used in Example 5. The 180° release force between the release sheet and adhesive layer in thus obtained patch was 0.3 N/40 mm.

### Example 10

A patch was prepared in the same manner as Example 5 except that perforations were provided such that the ratio between the length of junction (a) and the length of perforation (b) became a:b = 1:5.

### Example 11

A patch was prepared in the same manner as Example 5 except that perforations were provided such that the ratio between the length of junction (a) and the length of perforation (b) became a:b = 1:15.

### Example 12

A patch was prepared in the same manner as Example 5 except that a release sheet made of non-oriented polypropylene (thickness: 20 µm; bending resistance: 15 mm; tensile strength: 4 N/cm; elongation ratio: 240%) was used in place of the release sheet used in Example 5. The 180° release force between the release sheet and adhesive layer in thus obtained patch was 0.1 N/40 mm.

### Example 13

A patch was prepared in the same manner as Example 5 except that a release sheet made of polyethylene (thickness: 80 µm; bending resistance: 120 mm; tensile strength: 43 N/cm; elongation ratio: 310%) was used in place of the release sheet used in Example 5. The 180° release force between the release sheet and adhesive layer in thus obtained patch was 0.3 N/40 mm.

### Example 14

A patch was prepared in the same manner as Example 5 except that a release sheet made of oriented polypropylene (thickness: 50 µm; bending resistance: 80 mm; tensile strength: 35 N/cm; elongation ratio: 150%) was used in place of the release sheet used in Example 5. The 180° release force between the release sheet and adhesive layer in thus obtained patch was 0.05 N/40 mm.

### Example 15

A patch was prepared in the same manner as Example 5 except that a release sheet made of oriented polypropylene (thickness: 30 µm; bending resistance: 50 mm; tensile strength: 15 N/cm; elongation ratio: 130%) was used in place of the release sheet used in Example 5. The 180° release force between the release sheet and adhesive layer in thus obtained patch was 0.5 N/40 mm.

### Example 16

A patch was prepared in the same manner as Example 5 except that a release sheet made of silicone-treated oriented polypropylene (thickness: 40 µm; bending resistance: 45 mm; tensile strength: 26 N/cm; elongation ratio: 420%) was used in place of the release sheet used in Example 5. The 180° release force between the release sheet and adhesive layer in thus obtained patch was 0.01 N/40 mm.

Thus obtained patches were subjected to the following sensory evaluation tests:

### Test 4

As a tester, each of 20 healthy adult men and women evaluated the patches of Examples 5 to 9 and 12 to 16 in terms of the feel of use when applying them to aimed parts (knees) by pulling the patches leftward and rightward so as to tear and peel their release sheets according to the following criteria:
5: very easy to peel
4: easy to peel
3: relatively easy to peel
2: hard to peel
1: very hard to peel
Table 2 shows the average value of evaluation points obtained in each patch.

It was found that each of the patches of Examples 5 to 9 in which all of the bending resistance, tensile strength, and elongation ratio of the release sheet and the 180° release force between the release sheet and adhesive layer satisfied the preferred conditions mentioned above could easily tear and release the release sheet without turning up the release sheet at parts other than perforations, while yielding a favorable feel of use at the time of applying the patch.

### Test 5

As a tester, each of 20 healthy adult men and women evaluated the patches of Examples 5, 10, and 11 in terms of the easiness of tearing when tearing the release sheet by pulling the patches leftward and rightward according to the following criteria:
5: very easy to tear
4: easy to tear
3: relatively easy to tear
2: hard to tear
1: very hard to tear
Table 2 shows the average value of evaluation points obtained in each patch.

As shown in Table 2, it was found that each of the patches of Examples 5, 10, and 11 had a very high easiness of tearing, among which Examples 5 and 11 exhibited a particularly high easiness of tearing.

### Test 6

Each of the patches of Examples 5 to 9 and 12 to 16, with the release sheet attached thereto, was put into an aluminum foil package bag, which was then hermetically heat-sealed. After the aluminum foil package bag was stored for 1 month under the condition of 40°C, 75% RH, the patch was taken out from the package bag, and whether peeling occurred or not was visually evaluated according to the following criteria:
○: no peeling was seen at all between the adhesive layer and release sheet
X: peeling was partly seen between the adhesive layer and release sheet
Table 2 shows the results.

As shown in Table 2, no peeling of the release sheet was seen in any of the patches of Examples 5 to 9 when not in use.

Thus, it was found that a high releasability when in use and a high resistance to peel when not in use were achieved with a sufficiently favorable balance in the patches of Examples 5 to 11.

**TABLE 2**

| | Evaluation point(average value) | | |
|---|---|---|---|
| | Test4 (Feel of use) | Test5 (Easiness of tearing) | Test6 (Resistance to peel) |
| Example5 | 4.6 | 4.6 | ○ |
| Example6 | 4.5 | - | ○ |
| Example7 | 4.8 | - | ○ |
| Example8 | 3.9 | - | ○ |
| Example9 | 3.5 | - | ○ |
| Example10 | - | 3.5 | - |
| Example11 | - | 5.0 | - |
| Example12 | 1.3 | - | ○ |
| Example13 | 2.1 | - | ○ |
| Example14 | 1.8 | - | ○ |
| Example15 | 2.2 | - | ○ |
| Example16 | 2.6 | ― | × |

### Industrial Applicability

As explained in the foregoing, the present invention can yield a patch which can be applied be autifully and reliably in a short time when carrying out an operation of releasing a release sheet and an operation of applying the patch to an aimed part at the same time, while being hard to peel off even when attached to a joint, a face, or the like for a long period of time.

## Claims

1. A patch comprising a laminate having an expansible support and an adhesive layer laminated on said support; and a release sheet, attached onto said adhesive layer, having perforations formed with a predetermined interval;
wherein said laminate when formed into a test piece having a longer side of 20 cm and a shorter side of 5 cm exhibits a load of 0.98 to 14.71 N/5 cm in both longer and shorter side directions at the time of 50% elongation, and a 50% elongation recovery ratio of 50 to 95% in both longer and shorter side directions.

2. Apatch according to claim 1, wherein said release sheet has a bending resistance of 20 to 100 mm, a tensile strength of 4.90 to 29.42 N/cm, and an elongation ratio of 250 to 750%.

3. Apatch according to claim 1, wherein said release sheet exhibits a tear strength of 0.147 to 1.275 N/cm when torn along said perforations.

4. A patch according to claim 1, wherein, in said release sheet, the ratio between the length (a) of a junction between neighboring perforations and the length (b) of said perforation is a:b = 1:5 to 1:30.

5. Apatch according to claim 1, wherein said release sheet has a bending resistance of 20 to 100 mm, a tensile strength of 5 to 30 N/cm, and an elongation ratio of 250 to 750%.

6. Apatch according to claim 1, wherein said release sheet and said adhesive layer have a 180° release force of 0.03 to 0.4 N/40 mm therebetween.
